Europäisches Patentamt

European Patent Office

Office européen des brevets

(11) Publication number:

**0 235 727
A2**

# EUROPEAN PATENT APPLICATION

(21) Application number: 87102580.5

(22) Date of filing: 24.02.87

(51) Int. Cl.⁴: **C12Q 1/68** , //C12Q1/04

(30) Priority: 06.03.86 US 836787

(43) Date of publication of application:
09.09.87 Bulletin 87/37

(84) Designated Contracting States:
AT BE CH DE ES FR GB GR IT LI NL SE

(71) Applicant: Molecular Diagnostics, Inc.
400 Morgan Lane
West Haven, CT.06516(US)

(72) Inventor: Dattagupta, Nanibhushan
470 Prospect Street
New Haven, CT 06511(US)
Inventor: Rae, Peter M.M.
71 Ingram Street
hamfen, CT 06517(US)

(74) Representative: Jesse, Ralf-Rüdiger, Dr. et al
Bayer AG Konzernverwaltung RP
Patentabteilung
D-5090 Leverkusen 1 Bayerwerk(DE)

(54) Rapid dna test for microbes.

(57) A method of assaying a biological nucleic acid-containing sample for the presence of a particular microorganism, comprising
(a) immobilizing the sample,
(b) denaturing the nucleic acid,
(c) contacting the sample with an unfractionated, labelled, denatured whole genome DNA probe of the microorganism to be assayed for under hybridization conditions and
(d) assaying the hybridized nucleic acids by detecting the label.

EP 0 235 727 A2

## RAPID DNA TEST FOR MICROBES

### BACKGROUND OF THE INVENTION

The present invention relates to a method of assaying a biological nucleic acid-containing sample to detect the presence of a particular microorganism.

More particularly, the present invention concerns a very rapid and sensitive nucleic acid hybridization test to both identify and quantitate bacteria or other organisms that can be collected from biological samples.

Tests that are the present state-of-the-art involve probes that are particular RNAs or recombinant DNAs selected from cloned fragmented genomes or episomes on the basis of their representing some gene or other sequence having a desired set of properties. U.S. Patent 4,358,535 to Falkow et al, for example, described a probe specifically as being obtained from mRNA, a cDNA reverse transcript, or by cloning a gene or gene fragment. See also the 1985 overview article "Recombinant DNA and Other Direct Specimen Identification Techniques" by L. S. Tompkins, which was presented at a Symposium on Bacterial Identification Systems, and was published in Clinics in Laboratory Medicine, Vol. 5, pages 99-107. It is also possible to use the very highly repeated short circular DNA sequences found in specialized organelles of certain parasites, i.e., the kinetoplast (modified mitochondrion) DNA of Leishmania and related protozoa, to detect homologous sequences in animal tissue preparations infected by corresponding kinetoplast-containing protozoa (PCT/US83/01029).

It has been a widely held assumption that probe preparation for species specific DNA hybridization requires the identification and isolation of particular gene products or of short portions of a genome that have a base sequence that is unique or virtually so to the species of interest. Similarly, if the intent is to make a probe for DNAs of a certain group of related bacteria, it is expected that one will need to identify a gene product or a cloned segment of one of the relevant genomes that has the desired properties of cross-hybridization. It has been assumed that one must clone DNA and use plasmids or other recombinant DNAs as hybridization probes because hybridization studies involving unfractionated genomic DNA have historically been done to examine or demonstrate relatedness between or among species. (Johnson, J. L. (1984), Nucleic Acids in Bacterial Classification, pages 8-11, N. R. Krieg (ed.), Bergey's Manual of Systematic Bacteriology, Vol. 1. Williams and Wilkins, Baltimore). Emphasis in such studies has been on how well, rather than how poorly, two different genomic DNAs hybridize, so that it has been felt there must be too much DNA sequence homology between even distantly related bacterial species for uncloned DNAs to be useful probes.

Prior to this invention, a probe for the detection and identification of a species of microorganism has been a cloned DNA sequence of a few hundred to a few thousand nucleotides in length that is purified by recombinant DNA procedures from the bulk of a genome on the basis of some property such as demonstrable uniqueness to a species or a portion of a gene. There are disadvantages to this methodology that have been overcome by the present invention.

First, the generation, characterization and propagation of cloned DNA segments that have heretofore served as probes for the detection and identification of a species of microorganism required considerable effort by highly trained molecular biologists. Applicants have demonstrated herein that probe development and preparation by lengthy recombinant DNA procedures is unnecessary and even undesirable. Second, the extent to which a labelled unfractionated genomic DNA probe can hybridize to complementary sequences in sample unfractionated nucleic acid is two to three orders of magnitude greater than the extent to which a cloned small portion of the genome is able to and this results in a manyfold magnification of the hybridization signal and greater sensitivity, or more rapid detection. Third, while there are cloned-DNA-to-cellular-ribosomal-RNA (rRNA) hybridization procedures that exploit the abundance of rRNA in cells to acheive rapid hybridization and detection (e.g., the tissue culture mycoplasm contamination test commercialized by GenProbe, San Diego, California, U.S. A), the whole genome hybridization system of the present invention compares favorably in terms of number of detectable sequences per cell and has the advantages that (i) labelled genomic DNA probes can form signal-amplifying networks during hybridization that recombinant DNA probes cannot and (ii) the amount of genomic DNA in a cell is invariant, while the amount of rRNA can fluctuate considerably according to the physiological state of a cell.

## SUMMARY OF THE INVENTION

The present invention concerns a method of assaying a biological nucleic acid-containing sample for the presence of a particular microogranism, e.g., bacteria, fungi, virus or protozoa, comprising
(a) immobilizing the sample,
(b) denaturing the nucleic acid,
(c) contacting the sample with an unfractionated, labelled, denatured whole genome DNA probe of the microorganism to be assayed for under hybridization conditions and
(d) assaying the hybridized nucleic acids by detecting the label.

The present invention also concerns a kit for assaying a biological nucleic acid-containing sample for the presence of a particular microorganism comprising
(a) labelled whole genomic DNA from known microorganisms,
(b) immobilized reference nucleic acid samples on a solid support, the support being available for immobilization of the sample,
(c) hybridization reagents, and
(d) reagents for chemiluminescence detection.

In the above described kit, the hybridization reagent can contain the labelled whole genomic DNA from known microorganisms.

Hybridization reagents as described above can include a mixture of sodium chloride, sodium dodecyl sulfate, bovine serum albumin or nonfat milk and optionally formamide.

A novel feature of the present invention is that unfractionated bacterial or other whole genomic DNAs can be used as hybridization probes for the rapid detection and identification of nucleic acids in sample materials. The present invention is not limited by hybridization format, as it can incorporate a variety of sample preparation procedures, probe labelling protocols, and annealing and washing conditions. The use of all, or essentially all, the sequences in a genome as probe, rather than DNA that has been fractionated in some way such as by cloning, allows for rapid detection of a hybridization signal. This is in part because a preparation of DNA from cells that has not been depleted of any particular sequences (defined as genomic DNA) has the ability to hybridize more completely to a sample of homologous cellular DNA than does a portion of the same DNA.

Rapid signal detection in accordance with the present invention is also facilitated by networking of the labelled probe that results from the annealing of randomly terminated fragments that overlap in their content of complementary sequence. The purification and labelling of probe DNA involves essentially random shearing of the double-stranded molecules and nicking of the individual polynucleotide chains. The result of these shearings and nickings is that when probe DNA is denatured (made single-stranded) for hybridization with a sample DNA, the preparation is comprised of a population of overlapping complementary sequences. During the process of hybridization, some of the single-strands of probe DNA will anneal with sample DNA, and some will anneal with other probe sequences. Without wishing to be bound by any particular theory of operability, it is believed that such hybridization of randomly terminated probe sequences will result in duplexes having single-stranded ends that remain available for hybridization to sample DNA or more probe DNA. This means that molecules of probe DNA that anneal with sample DNA can also anneal with other molecules of probe DNA and so on, thus forming a network and amplifying the hybridization signal.

The surprising discovery of the present invention was that by adjusting parameters of the hybridization process, temperature, salt concentration and the presence of organic solvents applicant was able to define conditions of hybridization and stringency washing that allowed clear discrimination among purified DNA samples from a number of bacterial genera when they were probed as a set with a series of labelled preparations of the same unfractionated DNAs. Applicant then found that the procedure could be applied with a few modifications, i.e., composition of the hybridization solution, time of hybridization, etc., supra, to the identification and quantitation of DNA in crude call lysates.

## DETAILED DESCRIPTION OF THE INVENTION

The ability of the hybridization test to discriminate among organisms depends to a considerable extent on the conditions, or criteria, that are chosen for nucleic acid hybridization and for the post-hybridization washes. The conditions that can be manipulated to affect the quality of hybridization are (1) the concentration of salt in the hybridization and washing solutions, and (2) the temperature of the solutions. In general, the proportion of mismatched bases tolerated in a nucleic acid duplex decreases as the salt concentration is reduced and/or as the temperature is elevated. The stringency of hybridization is defined by these parameters, which can be manipulated to require perfect base pairing or to allow less than perfect matching over a range of values. This means that by adjusting the conditions, or stringency, one can achieve degrees of discrimination among closely and distantly related species. For example, it is possible in one mode of the present invention to distinguish only gram positive and gram negative bacteria and in another mode to discriminate members of the Enterobacteriaceae from other families of microorganism and in the most sensitive mode to identify a bacterium in this family as being, say, Escherichia coli, rather than Klebsiella pneumoniae.

As with hybridization systems that involve cloned probes or fractionated nucleic acids, the specificity of labelled genomic DNA hybridization to homologous sequences in a mixed sample can be enhanced by reduction of the interaction of the probe with any nucleic acids in the sample that may be related, but not truly complementary to the probe. This is accomplished by inclusion in pre-hybridization and/or hybridization steps of unlabelled known nucleic acids that will saturate in a mixed sample the DNA or RNA sequences that would interfere with a desired measurement by binding some of the labelled probe.

In the examples given hereinbelow, labelling of the probe is either by radioactivity, where detection of hybrid is by autoradiography of a sample membrane, or by the incorporation of biotin by nick translation where detection of hybrid is by an enzymatic color production at sites of probe accumulation. However, other probe labelling schemes are equally applicable to the present invention since there is no limiting read-out requirement.

The present invention can be applied to any situation where organisms or cells can be collected, concentrated and lysed, either in situ on a matrix such as nitrocellulose paper, or in suspension. Thus, the present invention can be applied not only to bacteria, but also to the detection and identification of other microorganisms. For example, viruses such as cytomegalovirus, herpes simplex and human T-lymphotropic virus, fungi such as Candida albicans which is the primary etiological agent of vaginitis and other mycotic infections and such pathogenic protozoa as Entamoeba histolytica, Plasmodium species responsible for malaria and Trichomonas vaginalis, just to name a few. A comprehensive discussion of medical microbiology is found in: Lennette, E. H., ed., Manual of Clinical Microbiology, 3rd Edition, American Society for Microbiology, Washington, 1980. The present invention is particulary useful in the diagnosis of urinary tract infections.

The following bacteria can lead to urinary tract infections (UTI):

Escherichia coli : Incidence from 60 to 90%. Most common UTI pathogen, in both cystitis (lower UTI) and pyelonephritis (kidney involvement). The majority of UTIs are caused by only a limited number of ca. 160 O-serotypes; these are 01, 02, 04, 06, 07, 09, 011, 018, 025, 039, 075; O-negative isolates are also frequently encountered.

Proteus spp.: Second most frequently encountered urinary pathogen; 5 to 20%. Can cause extremely severe kidney infection. Three species:

P. mirabilis (most common), P. morganii and P. retgeri . Serology complex, but not particularly relevant.

Klebsiella spp.: Incidence 3 to 10%. Somewhat arbitrarily divided into a number of species; K. pneumoniae most important in UTI.

Staphylococcus spp.: Two species relevant to UTI, S. saprophyticus and S. epidermidis. Incidence 4 to 20%. Once dismissed as a contaminant of urine specimens, these organisms have recently been found to be frequent causative agents.

Streptococcus spp.: S . faecalis most common; incidence 2 to 5%.

Other gram-negative rods:
These together account for a low percent of UTI; include Pseudomonas aeruginosa, Enterobacter aerogenes, E. hafnia , E. liquefaciens, Serratia marcescens, Citrobacter and Acinetobacter.

The aims of a test for urinary tract infections are threefold:

(1) determine if a sample is sterile or not (if a sample contains more than $10^4$ or $10^5$ cells per ml, depending on circumstances);

(2) if there is an infection, determine if it is associated with Gram positive or Gram negative bacteria;

(3) determine the particular species of bacterium responsible for the infection.

In one preferred embodiment, the material to be tested is collected as a small spot on a nitrocellulose membrane and bacteria or other organisms are lysed in situ by floating the membrane on a NaOH solution; this also has the effect of denaturing DNA and immobilizing it on the membrane at the spot. On the same or on an accompanying membrane are standards, which are spots of known amounts of denatured DNAs of organisms for which a fluid is being tested. After DNA denaturation, the membrane is treated to prepare it for hybridization.

The probe can be DNA from one or several species, depending on the anticipated complexity of the sample to be tested and the need to make simultaneous determinations. The probe can be labelled with radioisotopes or nonisotopically, and the labelling can be accomplished in vivo or in vitro. Different probes in a single mixture could be labelled in different ways so that the hybridization of more than one ω a complex sample of DNAs could be detected and identified individually, as could the hybridization of a single member of a mixture of probes.

Probe DNA can be prepared from, say, a culture of bacteria by standard methods that involve detergent lysis, organic solvent extraction, ethanol precipitation and dissolution of DNA in a buffered aqueous medium. As a result of this handling, the DNA from each cell is no longer in the form of one double-stranded molecule of chromosome size as it is in intact cells; rather, the purification process shears the DNA into smaller molecules of perhaps 100,000 base pairs (100 kb) average length. Thus, the bacterial chromosome is fragmented during purification and, while breaks generally produce 1/2, 1/4, 1/8, etc. -sized molecules, the sites of breakage are random with respect to DNA sequence.

Probe DNA labelling can be accomplished in a number of ways, some involving radioisotopes and some not. A sample of DNA that is to serve as probe is comprised of a very highly overlapping set of fragments of a genome; in the case of bacteria - (and viruses), genome is synonymous with the expression "chromosome-sized DNA molecule" since virtually all of the genetic information in a bacterium is contained in a single DNA molecule that comprises what is thought of as the bacterial chromosome. One labelling procedure that is demonstrably effective in the hybridization system is nick translation. This procedure involves treating a DNA sample with two enzymes, namely, DNase I and DNA polymerase. DNase I is an endonuclease that introduces single-stranded nicks essentially at random in the double-stranded DNA molecules; DNA polymerase then uses the nicks as points of initiation of DNA synthesis and incorporates deoxyribonucleoside triphosphates into polymeric DNA by displacing portions of the existing polynucleotide chains. The nicking by DNase I also results in a much reduced molecular length of DNA at the level of single-strands.

The speed and high sensitivity of hybridization (unambiguous detection of $10^4$ bacteria in a spot in a few hours) is achieved by using samples of unfractionated bacterial DNA as a labelled probe, rather than cloned small portions of a genome. Specificity of hybridization (unambiguous discrimination of bacterial species) is achieved by selecting a particular set of conditions for hybridization and filter washing.

A variety of methods can be used to label polynucleotide probes. It is preferable to have the label distributed along the length of the polynucleotide rather than clustered at one position. Several means for labelling are outlined below.

5-(3-Amino)allyldeoxyuridine triphosphate (AA-duTP) or its biotinylted derivative can be synthesized by the method of Langer et al, Proc. Nat'l. Acad. Sci., 78:6633 (1981) and can be introduced into double stranded DNA probes by nick translation with DNA polymerase. Probes with pendant amino groups can be reacted with any amine reactive labels.

The N-hydroxysuccinimide esters of 6-carboxyfluorescein, 4',5'-dimethoxy-6-carboxymethylfluorescein and their beta-galactosyl derivatives can be reacted directly with the nick translated probe. The activated dyes would be used in excess to maximize the incorporation of label and then uncoupled dye would be separated from the labelled DNA by a gel filtration method.

The labels with terminal amines on the bridge groups can be coupled to the nick translated DNA by means of bifunctional reagents such as dimethyladipimidate, hexamethylene diisocyanate, and p,p'-difluoro-m,m'-dinitro-phenylsulfone. The labels can be reacted with an excess of the bifunctional reagent followed by removal of the unreacted reagent. The activated label can be reacted with the nick translated probe.

Certain planar aromatic compounds intercalate between the base pairs of double stranded nucleic acids to form reversible complexes. Some intercalating agents can be coupled covalently to the polynucleotides by photolysis of the intercalation complexes. Examples of photoactivatable intercalating agents are 8-azidoethidium, 8-azidomethidium and furocoumarins such as angelicin. These intercalators can be modified with bridge arms containing functional groups. The 8-azidomethidium derivatives can be prepared as described by Hertzberg and Dervan, J. Am. Chem. Soc., 104:313 (1982) and Mitchell and Dervan, J. Am. Chem. Soc., 104:4265 (1972). The labels can be coupled directly to the modified intercalators. For example, the N-hydroxysuccinimide esters of 6-carboxyfluorescein or 4',5'-dimethoxy-6-carboxyfluorescein can be reacted with the intercalator derivatives to form amide linkages. Then these intercalator-label conjugates can be added to a polynucleotide probe and photolyzed. Alternatively, the modified intercalators can be photolyzed with polynucleotide probe and then coupled to the label compounds. 8-Azidomethidium can be coupled photolytically to single stranded nucleic acids through a nonintercalative mechanism (Balton and Kerns (1978) Nucl. Acids Res. , 5:4891). This means can be used to couple this intercalator derivative to single stranded DNA and RNA.

For the detection of RNA-DNA hybrids an antibody can be used. Immunogens for stimulating antibodies specific for RNA DNA hybrids can comprise homopolymeric or heteropolymeric polynucleotide duplexes (Kitagawa and Stollar, (1982) Mol. Immunol. 19:4131). The selected RNA DNA duplexes are adsorbed to a methylated protein; or otherwise linked to a conventional immunogenic carrier material, such as bovine serum albumin and injected into the desired host animal (Stollar (1980), Meth. Enzymol, 70:70). The antibody reagent can consist of whole antibodies, antibody fragments, polyfunctional antibody aggregates, or in general any substance comprising one or more specific binding sites from an antibody. The immunoglobulin source for the antibody reagent can be obtained in any available manner such as conventional antiserum and monoclonal techniques.

The test sample to be assayed can be any medium of interest and will usually be a liquid sample of medical, veterinary, environmental, nutritional, or industrial significance. Human and animal specimens and body fluids particularly can be assayed by the present method, including urine, blood (serum or plasma), milk, amniotic fluid cerebrospinal fluid, sputum, fecal matter, lung aspirates, throat swabs, genital swabs and exudates, rectal swabs and nasopharnygal aspirates. Where the test sample obtained from the patient or other source to be tested contains principally double stranded nucleic acids, such as contained in cells, the sample will be treated to denature the nucleic acids and if necessary first to release nucleic acids from cells. Denaturation of nucleic acids is preferably accomplished by heating in boiling water or alkali treatment (e.g., 0.1 N sodium hydroxide), which if desired, can simultaneously be used to lyse cells. Also, release of nucleic acids can, for example, be obtained by mechanical disruption (freeze/thaw, abrasion, sonication), physical/chemical disruption (detergents such as "TRITON", "TWEEN", sodium dodecylsulfate, alkali treatment, osmotic shock, or heat), or enzymatic lysis (lysozyme, proteinase K, pepsin). The resulting test medium will contain nucleic acids in single stranded form which can then be assayed according to the present hybridization method.

The present invention additionally provides a reagent system, i.e., reagent combination or means, comprising the essential elements required to conduct a desired assay method. The reagent system is presented in a commercially packaged form, as a composition or admixture where the compatability of the reagents will allow, in a test device configuration, or more usually as a test kit, i.e., a packaged combination of one or more containers, devices, or the like holding the necessary reagents, and usually including written instructions for the performance of assays. Reagent systems of the present invention include all configurations and compositions for performing the various hybridization formats described herein.

The reagent system will comprise
(1) labelled nucleic acid probes as described herein, and
(2) sample DNA immobilization membranes which can include preloaded reference and control DNA spots. A test kit form of the system can additionally include ancillary chemicals such as the components of the hybridization solution and denaturation agents capable of converting double stranded nucleic acids in a test sample into single stranded form. Preferably, there is included a chemical lysing and denaturing agent, e.g., alkali, for treating the sample to release single stranded nucleic acid therefrom.

The major advantages of this invention are as follows:

(1) The use of the entire genome of a bacterial species, for example, as a probe has decided advantages over the standard use of cloned segments of the same genome.

(a) The much greater extent of sequence homology between the probe and a homologous genome, where potentially all sequences can hybridize, gives a much stronger signal than does a hybridization involving homology between only a portion of a genome and a cloned segment as probe.

(b) The supply of probe DNA depends only on the growth of cells and a simple purification of the DNA from cell lysates; no recombinant DNA procedures are required.

(c) Quality control of probes involves only standard microbiological identification of bacteria, and simple physical characterization of DNA.

(2) The use of denatured DNA in crude cell lysates rather than purified DNA as the immobilized sample material gives another and unexpected amplification of hybridization signal insofar as applicant has observed that about five times fewer cells can be detected as expected from studies using purified DNAs. Further, because the sample to be probed is simply denatured DNA in an alkaline cell lysate that can be prepared by filtering or spotting cells on a filter, the preparation of the sample is neither complicated nor critical.

It is well known in the prior art that a bacterial cell contains several thousand copies of ribosomal RNA. When a whole cell lysate is prepared using conditions under which both RNA and DNA are preserved, all the nucleic acid-containing material can be immobilized for hybridization; similarly the RNA can be purified and immobilized alone - (Thomas, P. S. "Hybridization of Denatured RNA and Small DNA Fragments Transferred to Nitrocellulose Paper", Proc. Nat'l. Acad. Sci., U.S.A, 77, 5201-5205 (1980)). When whole genome DNA is used as probes, the RNA molecules will be detectable by hybridization to the ribosomal RNA gene portions of the probe DNA. The advantages of this use of whole genome probes are that

(i) the abundant ribosomal RNA can be rapidly detected by hybridization to a labelled probe without the necessity of the prior generation of cloned ribosomal RNA genes by recombinant DNA procedures, and

(ii) in addition to the rapidity of hybridization detection afforded by the abundance of rRNA in a cell, networking of genomic probe DNA during hybridization results in an additional amplification of the hybridization signal, as discussed hereinabove.

The following non-limiting example describes one method in which the invention can be applied to the diagnosis of urinary tract infections.

Example

(1) Concentrate cells, if necessary, by centrifugation in order to be able to spot or filter 1 to 100 μl of suspension onto a nitrocellulose sheet. Alternatively, filter 1 to 100 ml of urine or other fluid directly through a spot on nitrocellulose.

(2) Wet nitrocellulose on 0.5 M NaOH to lyse bacteria and denature DNA in situ . Neutralize in 1.0 M Tris-HCl, pH 7.5, then rinse filter in 1.5 M NaCl, 0.5 M Tris, pH 7.5.

(3) Blot filter, then bake at 80°C under vacuum for 1/2 to 2 hours.

(4) Prehybridize the filter in a salt/protein/SDS solution for 1/2 to several hours at 68°C, then add denatured probe DNA that had been biotinylated by nick-translation using Bethesda Research Laboratories ("BRL"), Gaithersburg, Maryland, U.S.A. 20877, biotin-11-dUTP as one of the precursor triphosphates. Hybridize at 68°C for at least one hour.

(5) Wash the filter for a few minutes each in a series of salt/SDS solutions at room and elevated temperatures to remove unbound probe and dissociate unstable hybrids.

(6) Saturate the filter with protein solution, then add BRL streptavidin. After washing out the unbound streptavidin, add BRL biotinylated poly-(alkaline phosphatase). After further washes, incubate the filter in a solution containing nitro-blue tetrazolium and 5-bromo-4-chloro-3-indolyl phosphate, both from BRL. Insoluble color develops on the filter at sites of hybridization.

Using genomic DNAs of bacterial species as probes, with labelling by nick-translation using biotinylated nucleotides and with hybrid detection using the BRL streptavidin/polyalkalkine phosphatase kit, applicant has been able to:

(1) readily detect 1 ng of purified DNA in a spot on nitrocellulose;

(2) detect $10^4$ bacterial cells lysed in situ in a spot on nitrocellulose ($10^4$ genomes = approximately 50 pg);

(3) clearly distinguish DNAs of the several different genera of bacteria involved in urinary tract infections;

(4) condense the preparation, hybridization and detection of DNA to a total of 3 hours (for probe hybridization to tens of ng homologous DNA).

It will be appreciated that the instant specification and claims are set forth by way of illustration and not limitation, and that various modifications and changes may be made without departing from the spirit and scope of the present invention.

**Claims**

1. A method of assaying a biological nucleic acid-containing sample for the presence of a particular microorganism, comprising

(a) immobilizing the sample,

(b) denaturing the nucleic acid,

(c) contacting the sample with an unfractionated, labelled, denatured whole genome DNA probe of the microorganism to be assayed for under hybridization conditions and

(d) assaying the hybridized nucleic acids by detecting the label.

2. A method according to claim 1, wherein the microorganism is selected from the group consisting of bacteria, fungi, viruses and protozoa.

3. A method according to any of claims 1 and 2, wherein the microorganism is responsible for a urinary tract infection.

4. A method according to any of claims 1 to 3, wherein the microorganism is selected from the group consisting of Escherichia coli, Proteus spp., Klebsiella spp., Staphylococcus spp., Streptococcus spp., Pseudomonas spp. and Lactobacillus spp.

5. A method according to any of claims 1 to 4, wherein the labelling is conducted in a whole living cell in a cell lysate or with extracted nucleic acid.

6. A method according to any of claims 1 to 5, wherein the label is selected from the group consisting of protein binding ligands, haptens, antigens, fluorescent compounds, dyes, radioactive moieties and enzymes.

7. A method according to claim 6, wherein the protein binding ligand is a biotin-containing moiety.

8. A method according to any of claims 1 to 7, wherein the detection of the label is conducted by chemiluminescence or by an enzyme reaction.

9. A method according to any of claims 1 to 8, wherein the immobilization is carried out by chemical reaction or physical adsorption by use of a support selected from the group consisting of filters, solid sheets and beads.

10. A method according to claim 1, wherein said labelling is carried out by photochemically reacting a DNA binding ligand with nucleic acids in the probe.